(19) Europäisches Patentamt
European Patent Office
Office européen des brevets

(11) Numéro de publication : 0 077 279 B1

# (12) FASCICULE DE BREVET EUROPÉEN

(45) Date de publication du fascicule du brevet : 07.09.88

(51) Int. Cl.[4] : C 07 C 45/68, C 07 C 47/565, C 07 C 47/575, C 07 C 47/58

(21) Numéro de dépôt : 82420136.2

(22) Date de dépôt : 28.09.82

## (54) Procédé de préparation d'orthohydroxybenzaldéhydes.

(30) Priorité : 09.10.81 FR 8119209

(43) Date de publication de la demande : 20.04.83 Bulletin 83/16

(45) Mention de la délivrance du brevet : 07.09.88 Bulletin 88/36

(84) Etats contractants désignés : AT BE CH DE GB IT LI NL

(56) Documents cités :
FR-A- 2 428 625
JOURNAL OF THE CHEMICAL SOCIETY PERKIN-TRANS., no. 9, 1980, pages 1862-1865. G. CASIRAGHI et al.: "Selective reactions between phenols and formaldehyde. A novel route to salicylaldehydes"
CHEMICAL ABSTRACTS, vol. 89, 1978, page 547, no. 163270q, Columbus, Ohio, USA

(73) Titulaire : RHONE-POULENC CHIMIE
25, quai Paul Doumer
F-92408 Courbevoie Cédex (FR)

(72) Inventeur : Formanek, Karel
3 rue de la Sarrazinière
F-69360 Serezin-du-Rhône (FR)

(74) Mandataire : Vignally, Noel et al
RHONE-POULENC INTERSERVICES Service Brevets Chimie Centre de Recherches de Saint-Fons B.P. 62
F-69192 Saint-Fons Cedex (FR)

EP 0 077 279 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet un procédé de préparation d'orthohydroxybenzaldéhydes et notamment d'aldéhyde salicylique.

L'aldéhyde salicylique et ses dérivés de substitution sont des composés chimiques présentant un très grand intérêt industriel. Ils sont en effet utilisés comme intermédiaires en synthèse organique ou pour leurs propriétés propres. Ainsi l'aldéhyde salicylique est employé comme matière première pour la fabrication de la coumarine, en parfumerie et comme additif dans l'essence.

Un des procédés utilisés industriellement pour préparer sélectivement les ortho-hydroxybenzaldéhydes consiste à oxyder les alcools ortho-hydroxybenzyliques et notamment le saligénol par l'oxygène en milieu aqueux alcalin en présence d'un métal noble tel que le palladium et le platine, éventuellement en présence d'un promoteur comme le bismuth, cf. brevets français 1 337 243 et 2 305 420. Les alcools ortho-hydroxybenzyliques sont eux-mêmes obtenus par condensation du formaldéhyde ou d'un générateur de formaldéhyde avec un borate d'aryle pour former des borates de l'alcool ortho-hydroxybenzylique que l'on décompose pour libérer l'alcool correspondant (cf. brevets français 1 329 945 et 2 430 928). L'enchaînement de ces deux étapes de condensation et d'oxydation constitue un procédé indirect d'introduction d'un groupe aldéhyde en position ortho du groupe hydroxyle d'un phénol. L'industrie est à la recherche de procédés permettant d'obtenir directement des orthohydroxybenzaldéhydes par réaction du formaldéhyde ou d'un générateur de formaldéhyde avec le phénol ou un phénol substitué. C'est pour répondre à ce besoin qu'on a proposé dans la demande de brevet français N° 77 24 727 publiée sous le N° 2 361 327 un procédé direct de préparation des ortho-hydroxybenzaldéhydes par réaction des phénols présentant au moins une position ortho libre avec du formaldéhyde en présence de chlorure stanneux ou de chlorure stannique et de bases azotées prises dans le groupe des amines primaires, secondaires ou tertiaires et des bases hétérocycliques (pyridines et dérivés de substitution). Dans la demande de brevet français N° 79 15 162 publiée sous le N° 2 428 625 on a proposé un procédé analogue dans lequel on fait appel, à titre de catalyseur, à des dérivés du chrome-III et du fer-III éventuellement activés par une base tertiaire hétérocyclique (pyridine) ou une amine tertiaire aromatique ou aliphatique.

L'article de G. CASIRAGHI et al. dans « Journal of the Chemical Society Perkin-trans », n° 9, 1980, pages 1862 à 1865, décrit l'utilisation d'halogénures d'étain-II et IV, de fer-III et à un moindre degré d'aluminium et de cuivre-II comme catalyseurs, en présence d'amine tertiaire et en opérant dans un solvant aprotique et faiblement électro-donneur.

Le résumé n° 163 270q des Chemical Abstracts volume 89 (1978) indique que l'on peut faire réagir un orthométhoxy ou orthoéhtoxyphénol avec le formaldéhyde en présence d'aluminium. Dans cette réaction la formylation se réalise en position para par rapport à la fonction phénolique. En définitive l'art antérieur enseigne la condensation directe du formaldéhyde avec un phénol en ortho-hydroxybenzaldéhyde en présence de dérivés de métaux qui n'apparaissent liés par aucun trait commun puisqu'ils appartiennent à des groupes différents de la classification périodique des éléments : groupe 6b pour le chrome, 8 pour le fer et 4a pour l'étain.

Il a maintenant été trouvé que d'autres métaux appartenant à d'autres groupes de la classification périodique des éléments que les métaux précités et d'autres bases, au sens de Lewis, que les bases azotées, permettent d'obtenir des ortho-hydroxybenzaldéhydes directement par réaction du formaldéhyde avec le phénol ou un phénol substitué.

Plus particulièrement la présente invention a pour objet un procédé de préparation d'orthohydrobenzaldéhydes de formule générale :

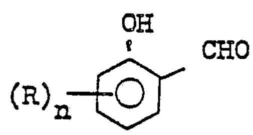

(I)

dans laquelle :

R représente un atome d'hydrogène, un radical alkyle comportant de 1 à 10 atomes de carbone, un radical alkyloxy comportant de 1 à 10 atomes de carbone, un groupe hydroxyle, un atome d'halogène, un groupe formyle ou deux groupes R placés sur deux atomes de carbone vicinaux forment ensemble et avec les atomes de carbone qui les portent un cycle benzénique ;

n est un nombre entier de 1 à 3

par réaction d'un polyformaldéhyde avec un phénol de formule générale :

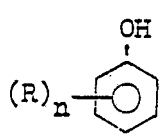

(II)

dans laquelle R et n ont la signification donnée ci-avant, dont l'une au moins des deux positions en ortho du groupe hydroxyle est libre, en présence d'un catalyseur métallique et éventuellement d'une base,

caractérisé en ce qu'on utilise comme catalyseur un dérivé du titane-IV.

Il est surprenant que le titane qui appartient au groupe 4b de la classification périodique (Handbook of Chemistry and Physics 53ème Edition 1972-73 publié par The Chemical Rubber C°) présentent une activité comparable à celle du chrome et du fer pour la réaction considéré alors que des métaux plus proches de ces derniers tels que le vanadium, le molybdène ou le nickel n'ont qu'un pouvoir catalytique médiocre, voire même mauvais.

Dans les formules (I) et (II), R représente de préférence un atome d'hydrogène, un radical alkyle inférieur, c'est-à-dire un radical comportant de 1 à 4 atomes de carbone tel que les radicaux méthyle, éthyle, n-propyle, isopropyle, n-butyle ou isobutyle, un radical alkyloxy inférieur comme les radicaux méthoxy ou éthoxy, ou un atome de chlore et n est de préférence égal à 1.

Parmi les phénols de formule (II) on peut citer le phénol, le p-crésol,le m-crésol, le p-chlorophénol, le métachlorphénol, le gaïacol, le β-naphtol, l'hydroquinone, la p-vanilline.

Le procédé selon la présente invention permet de préparer des ortho-hydroxybenzaldéhydes tels que le salicylaldéhyde, le dihydroxy-2,5 benzaldéhyde, l'hydroxy-2 méthyl-4 benzaldéhyde, l'hydroxy-2 méthyl-6 benzaldéhyde, l'hydroxy-2 méthyl-5 benzaldéhyde, l'hydroxy-2 méthoxy-3 benzaldéhyde, l'hydroxy-2 chloro-5 benzaldéhyde, l'hydroxy-2 naphtylaldéhyde, de diformyl-4,6 gaïacol.

Les dérivés du titane auxquels on fait appel pour la mise en œuvre du procédé selon l'invention sont des composés solubles dans le milieu réactionnel et susceptibles de former des complexes avec le phénol mis en œuvre et le formaldéhyde dans les conditions de la réaction. On peut utiliser notamment des sels d'acides minéraux, des alcoolates, des phénolates, des sels d'acides organiques carboxyliques ou sulfoniques, des complexes dérivés de composés donneurs de doublets électroniques. Parmi les composés du titane qui peuvent être utilisés on peut citer à titre non limitatif les ortho-titanates d'alcoyle ou d'aryle tels que les titanates de méthyle, d'éthyle, de n-propyle, de t-butyle, de phényle, de tolyte ; le lactate de di-isopropyloxytitane-IV ; des halogénures de titane comme $TiCl_4$ ; le stéarate de titane-IV, l'adipate de titane-IV ; les chélates dérivés de composés β-dicarbonylés comme les β-dicétones, les β-céto-aldéhydes et les β-cétoesters comme l'acétylacétonate de titane-IV ; des complexes comme le dichloro-dicyclopentadiényl-titane-IV ou le tetrakis (diméthylamino) titane.

Les bases utilisées comme activateur du catalyseur dans le procédé selon l'invention sont choisies parmi les composés donneurs de doublets électroniques dont le pouvoir complexant vis-à-vis du dérivé métallique considéré est inférieur à celui du substrat phénolique mis en œuvre. La nature de la base est donc déterminée par la nature du métal et du substrat phénolique. On fait appel notamment à des bases azotées tertiaires et en particulier à des bases tertiaires azotées hétérocycliques telles que le pyridine et ses dérivés de substitution (α-picoline, β-picoline) et leurs N-oxydes. Un autre groupe de bases de LEWIS convenant tout particulièrement bien à la mise en œuvre du procédé selon l'invention est constitué par les phosphines, les arsines et les stibines et les phosphotriamides. Les trialcoyl- et triarylphosphines sont utilisées de préférence. On peut citer notamment la triméthylphosphine, la triéthylphosphine, la tri-n-propylphosphine, la tri-n-butylphosphine, la tricyclohexylphosphine, la triphénylphosphine, la tritolyl-phosphine. Comme exemple de phosphotriamides on peut nommer l'hexaméthylphosphotriamide (HMPT) et l'hexaéthylphosphotriamide.

La réaction des phénols de formule (II) avec le formaldéhyde est conduite de préférence au sein d'un solvant organique inerte dans les conditions réactionnelles choisies. Comme solvant on utilise des liquides organiques aprotiques, peu ou pas polaires, parmi lesquels on peut citer des hydrocarbures aliphatiques ou cycloaliphatiques saturés (hexane, cyclopentane, cyclohexane) ou aromatiques (benzène, toluène) ; des hydrocarbures halogénés (chlorobenzène) ; des esters comme les acétates de butyle, d'isopropyle, d'amyle, de cyclohexyle ; des éthers comme l'oxyde de phényle ou de dioxanne. La concentration du substrat phénolique dans le solvant peut varier dans de larges limites. Ainsi elle peut être comprise entre 0,001 mole et 4 moles de phénol par litre de solvant et de préférence entre 0,01 et 2 moles par litre.

Le formaldéhyde est utilisé sous forme de polyformaldéhydes linéaires de degré de polymérisation indifférent. Ainsi on fait appel de préférence au paraformaldéhyde qui comporte entre 8 et 100 motifs $(CH_2O)$.

La quantité de catalyseur exprimée en atomes-grammes d'ions métalliques par mole de phénol peut varier dans de larges limites. En général une quantité comprise entre 0,0001 et 1 atome-gramme d'ion métallique par mole de phénol et de préférence entre 0,001 et 0,1 atome-gramme d'ion métallique convient bien. On peut toutefois sortir de ces valeurs limites sans que cela se traduise par des avantages particuliers.

La quantité de polyformaldéhyde exprimée en moles de formaldéhyde HCHO par mole de phénol peut, elle aussi, varier dans de larges limites. Bien que l'on puisse engager au moins la quantité théorique de formaldéhyde, c'est-à-dire 1 mole de HCHO par mole de phénol il est préférable d'opérer avec un excès de formaldéhyde. Ainsi le rapport molaire HCHO/phénol est de préférence au moins égal à 1. Il n'est pas nécessaire que ce rapport dépasse 10.

La quantité de base mise en œuvre, exprimée en mole par mole de substrat phénolique, dépend dans une large mesure de la nature de la base, de la nature du substrat phénolique et de la nature du catalyseur. De façon générale la quantité de base peut varier de 0,0001 à 1,5 mole par mole de phénol et, de préférence, de 0,01 à 1,2 mole par mole de phénol. On a constaté plus particulièrement que les

phosphines sont actives pour des quantités inférieures à celles des bases azotées. Ainsi il est généralement suffisant de mettre en œuvre une quantité de phosphine comprise entre 0,01 et 0,2 mole par mole de phénol tandis qu'il est préférable que la quantité de base azotée tertiaire soit comprise entre 0,2 et 1 mole par mole de phénol.

La température de la réaction peut varier de 120 à 250 °C et de préférence de 150 à 200 °C. Le procédé est conduit de préférence sous la pression autogène des réactifs pour éviter les pertes de formaldéhyde qui est gazeux aux températures mises en œuvre. Le recours à des pressions supérieures à la pression autogène ne se traduirait par aucun avantage. En pratique on préfère opérer en présence d'un gaz inerte tel que l'azote ou l'argon.

Les exemples suivants illustrent l'invention et montrent comment elle peut être mise en pratique. Dans les exemples on a utilisé comme source de formaldéhyde un polyoxyméthylène contenant 97,5 % en poids de HCHO.

## Exemple 1

Dans un tube en verre de 30 ml de contenance, on charge sous argon et dans l'ordre :
— un mélange solide et brassé préalablement à la spatule de :

| | |
|---|---|
| phénol | 471 mg ( 5,0 mM) |
| polyoxyméthylène | 61,5 mg (20,0 mM) |
| tétrabutoxyde de titane | 34 mg ( 0,1 mM) |

— 159 mg de pyridine soit 2 mM
— 9,65 g (11,0 ml) d'ortho-xylène.

Le tube est scellé avec les précautions d'usage, introduit dans sa gaine métallique de protection et placé dans un four agité pendant deux heures à 170 °C.

Après refroidissement à l'eau, on constate que la solution hétérogène verte est devenue presque homogène et marron foncé.

On ouvre le tube, rince par 10 ml d'o-xylène et analyse directement la solution réactionnelle par chromatographie en phase vapeur (étalon interne heptadécane).

Les résultats sont les suivants :

| | |
|---|---|
| taux de transformation du phénol | 73 % |
| rendement en aldéhyde salicylique par rapport au phénol transformé (RT) | 74 % |

On note également la présence d'ortho-méthoxyméthylphénol (RT 9 %) et de traces de saligénol.

## Exemples 2 et 3

Dans un autoclave en acier inoxydable de 250 ml on charge :

| | |
|---|---|
| phénol | 2,36 g |
| chlorobenzène | 125 ml |
| polyformaldéhyde | 3,9 g |
| pyridine | 1,8 g |
| tétraphénoxyde de titane | 0,32 g |

L'autoclave est fermé, purgé à l'azote puis on injecte 1 bar d'azote. Son contenu est porté à 190 °C et maintenu une heure dans ces conditions sous agitation. La masse réactionnelle est ensuite analysée comme à l'exemple 1. On a obtenu les résultats suivants :

| | |
|---|---|
| Taux de transformation du phénol (TT) | 80 % |
| Rendement en salicylaldéhyde par rapport au phénol transformé (RT) | 76 % |
| RT en o-méthoxyméthylphénol | 10 % |

On a répété l'exemple précédent sous une pression d'azote de 15 bars au lieu de 1 bar. Les résultats ont été les suivants :

| | |
|---|---|
| TT phénol | 81 % |
| RT salicylaldéhyde | 73 % |
| RT o-méthoxyméthylphénol | 7 % |

## Exemples 4 à 13

On a reproduit l'exemple 1 dans les conditions et avec les résultats consignés dans le tableau suivant :

| EX. | SOLVANT (en ml) | PHENOL (en mM) | FORMALDEHYDE (en g) | BASE en g | CATALYSEUR (en g) | T en °C | DUREE en H | TT-PHENOL % | RT-AS(1) % |
|---|---|---|---|---|---|---|---|---|---|
| 4 | chlorobenzène (50) | 5 | 0,62 | pyridine (0,4) | tetrakis(diméthylamino)titane (0,015) | 150 | 4 | 51 | 27 |
| 5 | acétate de n-butyle (12,5) | 5 | 0,31 | pyridine (0,4) | tetratolyloxyde de titane (0,024) | 170 | 2 | 48 | 37 |
| 6 | idem | idem | 1,55 | idem | idem | idem | idem | 81 | 41 |
| 7 | chlorobenzène (11) | idem | 0,62 | pyridine (0,46) | tetraphénoxyde de titane (0,046) | idem | 1,5 | 67 | 55 |
| 8 | idem | idem | idem | HMPT (0,41) | idem (0,042) | idem | 1,5 | 83 | 48 |
| 9 | idem | idem | idem | N-oxyde de pyridine (0,22) | idem | idem | 1,5 | 90 | 49 |

(1) RT-AS = rendement en salicylaldéhyde par rapport au phénol transformé.

| EX. | SOLVANT (en ml) | PHENOL (en mM) | FORMALDEHYDE (en g) | BASE en g | CATALYSEUR (en g) | T en °C | DUREE en H | TT-PHENOL % | RT-AS(1) % |
|---|---|---|---|---|---|---|---|---|---|
| 10 | chlorobenzène (16,5) | idem | idem | néant | dilactate du diisopropoxyde de titane (0,017) | 200 | 1 | 86 | 35 |
| 11 | idem | idem | idem | triphényl-phosphine (0,44) | idem | idem | idem | 51 | 57 |
| 12 | chlorobenzène (10,5) | idem | idem | triphényl-phosphine (0,07) | tetraphénoxyde de titane (0,038) | 170 | 1,5 | 87 | 56 |
| 13 | chlorobenzène (11) | idem | idem | pyridine (0,4) | dichlorodicyclopentadiényl-titane (0,004) | 230 | 0,5 | 85 | 54 |

(1) RT-AS = rendement en salicylaldéhyde par rapport au phénol transformé.

Exemple 14

En opérant comme à l'exemple 1 on a formylé le gaïacol dans les conditions et avec les résultats consignés ci-après :

| | |
|---|---|
| gaïacol | 5 mM |
| o-xylène | 11 ml |
| poly-formaldéhyde | 0,62 g |
| pyridine | 0,4 g |
| tétrabutoxyde de titane | 0,034 g |
| température | 170 °C |
| durée | 1h30 mn |
| TT gaïacol | 56 % |
| RT en hydroxy-2-methoxy-3 benzaldéhyde | 65 % |

Exemple 15

On a reproduit l'exemple 3 en remplaçant le phénol par 50 mM de métacrésol, dans les conditions suivantes :

| | |
|---|---|
| chlorobenzène | 100 ml |
| polyformaldéhyde | 6,2 g |
| pyridine | 4 g |
| tétraphénoxyde de titane | 0,21 g |
| température | 170 °C |
| durée | 2 h |

On a obtenu les résultats ci-après :

| | |
|---|---|
| TT m-crésol | 92 % |
| RT hydroxy-2-méthyl-4 benzaldéhyde | 30 % |
| RT hydroxy-2-méthyl-6-benzaldéhyde | 10 % |

Exemple 16

On a répété l'exemple 14 après avoir remplacé le m-crésol par le p-crésol et chargé 4,6 g de paraformaldéhyde au lieu de 6,2 g. On a obtenu les résultats suivants :

| | |
|---|---|
| TT p-crésol | 82 % |
| RT hydroxy-2-méthyl-5 benzaldéhyde | 47 % |

Exemple 17

On a reproduit l'exemple 14 après avoir remplacé le m-crésol par le p-chlorophénol et chargé 110 ml de chlorobenzène au lieu de 100 et 3,8 g de pyridine au lieu de 4 g. La température de réaction a été portée à 180 °C. Cette opération a fourni les résultats suivants :

| | |
|---|---|
| TT du chlorophénol | 33 % |
| RT en hydroxy-2-chloro-5 benzaldéhyde | 74 % |

Exemple 18

On a reproduit l'exemple 3 en remplaçant le phénol par 50 mM de formyl-4 gaïacol, dans les conditions suivantes :

| | |
|---|---|
| chlorobenzène | 110 ml |
| polyformaldéhyde | 7,6 g |
| triphénylphosphine | 0,12 g |
| tétrabutoxyde de titane | 0,34 g |
| température | 190 °C |
| durée | 1 h 30 |

On a obtenu les résultats suivants :

| | |
|---|---|
| TT formylgaïacol | 34 % |
| RT diformyl-4,6 | 26 % |

## Revendications

1. Procédé de préparation d'ortho-hydroxybenzaldéhydes de formule générale :

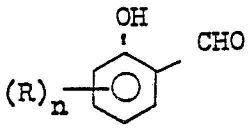

(I)

dans laquelle :

R représente un atome d'hydrogène, un radical alkyle comportant de 1 à 10 atomes de carbone, un radical alkyloxy comportant de 1 à 10 atomes de carbone, un groupe hydroxyle, un atome d'halogène, un groupe formyle ou deux groupes R placés sur deux atomes de carbone vicinaux forment ensemble et avec les atomes de carbone qui les portent un cycle benzénique ;

n est un nombre entier de 1 à 3

par réaction d'un polyformaldéhyde avec un phénol de formule générale :

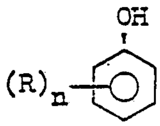

(II)

dans laquelle R et n ont la signification donnée ci-avant, dont l'une au moins des deux positions en ortho du groupe hydroxyle est libre, en présence d'un catalyseur métallique et éventuellement d'une base, caractérisé en ce que l'on utilise comme catalyseur un dérivé du titane-IV.

2. Procédé selon la revendication 1, caractérisé en ce que le dérivé du titane est un composé soluble dans le milieu réactionnel et susceptible de former des complexes avec le phénol mis en œuvre et le formaldéhyde dans les conditions de la réaction.

3. Procédé selon la revendication 2 caractérisé en ce que le catalyseur métallique est un sel d'acides minéraux, un alcoolate, un phénolate, un sel d'acides carboxyliques ou sulfoniques, un complexe du titane avec un composé donneur de doublet électronique.

4. Procédé selon la revendication 3, caractérisé en ce que le catalyseur métallique est pris dans le groupe formé par le tétraphénoxyde de titane, le tétrabutoxyde de titane, le tétratolyloxyde de titane, le dichlorodicyclopentadiényltitane, le tétrakis (diméthylamino) titane.

5. Procédé selon l'une quelconque des revendications 1 à 4, caractérisé en ce que la quantité de catalyseur exprimée en atomes-grammes d'ions métalliques par mole de phénol est comprise entre 0,0001 et 1 atome-gramme d'ion métallique par mole de phénol.

6. Procédé selon l'une quelconque des revendications 1 à 5 caractérisé en ce que le procédé est conduit en présence d'une base choisie dans le groupe des bases azotées tertiaires et de leurs N-oxydes, des phosphines, des arsines, des stibines et des phosphotriamides.

7. Procédé selon la revendication 6 caractérisé en ce que la base est la pyridine, la triphénylphosphine, la N-oxypyridine ou l'hexaméthylphosphotriamide.

8. Procédé selon l'une quelconque des revendications 1 à 7, caractérisé en ce que la quantité de base mise en œuvre, exprimée en mole par mole de substrat phénolique varie de 0,001 à 1,5 mole par mole de phénol.

9. Procédé selon l'une quelconque des revendications 1 à 8, caractérisé en ce que le procédé est conduit au sein d'un solvant organique inerte aprotique, non ou faiblement polaire.

10. Procédé selon la revendication 9, caractérisé en ce que le solvant est choisi dans le groupe formé par les hydrocarbures aliphatiques et cycloaliphatiques saturés, les hydrocarbures aromatiques, les hydrocarbures halogénés, les esters et les éthers.

11. Procédé selon l'une quelconque des revendications 1 à 8, caractérisé en ce que les phénols mis en œuvre sont le phénol, le métacrésol, le paracrésol, le gaïacol, le p-chlorophénol, la p-vanilline.

## Claims

1. Process for the preparation of ortho-hydroxybenzaldehydes having the general formula :

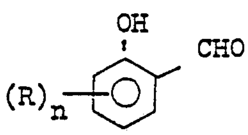

(I)

in which

R represents a hydrogen atom, an alkyl radical having 1 to 10 carbon atoms, an alkoxy radical having 1 to 10 carbon atoms, a hydroxyl group, a halogen atom or a formyl group, or two R groups, attached to two adjacent carbon atoms, form, together and with the carbon atoms carrying them, a benzene ring, and

n is an integer from 1 to 3,

by the reaction of a polyformaldehyde with a phenol having the general formula :

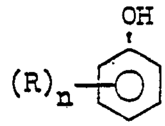

(II)

in which R and n have the meaning given above and in which at least one of the two positions ortho to the hydroxyl group is free, in the presence of a metal catalyst and, if appropriate, of a base, characterized in that the catalyst used is a derivative of titanium (IV).

2. Process according to Claim 1, characterized in that the derivative of titanium is a compound soluble in the reaction medium and capable of forming complexes with the phenol used and formaldehyde under the reaction conditions.

3. Process according to Claim 2, characterized in that the metal catalyst is a salt of inorganic acids, an alcoholate, a phenolate, a salt of carboxylic or sulphonic acids, or a complex of titanium with an electron pair donor compound.

4. Process according to Claim 3, characterized in that the metal catalyst is taken from the group formed by titanium tetraphenoxide, titanium tetrabutoxide, titanium tetratolyl oxide, dichlorodicyclopentadienyl titanium and tetrakis-(dimethylamino)-titanium.

5. Process according to any one of Claims 1 to 4, characterized in that the quantity of catalyst, expressed in gram-atoms of metal ions per mol of phenol, lies between 0.0001 and 1 gram-atom of metal ion per mol of phenol.

6. Process according to any one of Claims 1 to 5, characterized in that the process is performed in the presence of a base selected from the group of tertiary nitrogen-containing bases and their N-oxides, phosphines, arsines, stibines and phosphotriamides.

7. Process according to Claim 6, characterized in that the base is pyridine, triphenyl-phosphine, N-oxypyridine or hexamethylphosphotriamide.

8. Process according to any one of Claims 1 to 7, characterized in that the quantity of base used, expressed as mol per mol of phenolic substrate, varies from 0.001 to 1.5 mol per mol of phenol.

9. Process according to any one of Claims 1 to 8, characterized in that the process is carried out in an aprotic inert organic solvent medium having little or no polarity.

10. Process according to Claim 9, characterized in that the solvent is chosen from the group formed by saturated aliphatic and cycloaliphatic hydrocarbons, aromatic hydrocarbons, halogenated hydrocarbons, esters and ethers.

11. Process according to any one of Claims 1 to 8, characterized in that the phenols used are phenol, metacresol, para-cresol, guaiacol, p-chlorophenol or p-vanillin.

**Patentansprüche**

1. Verfahren zur Herstellung von ortho-Hydroxybenzaldehyden der allgemeinen Formel

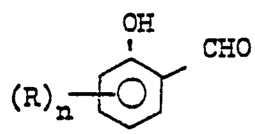

(I)

worin

R ein Wasserstoffatom, einen Alkylrest mit 1 bis 10 Kohlenstoffatomen, einen Alkyloxyrest mit 1 bis 10 Kohlenstoffatomen, eine Hydroxylgruppe, ein Halogenatom, eine Formylgruppe darstellt oder zwei Gruppen R, die an zwei benachbarten Kohlenstoffatomen stehen, bilden zusammen mit den sie tragenden Kohlenstoffatomen einen Benzolring ;

n eine ganze Zahl von 1 bis 3 ist,

durch Reaktion eines Polyformaldehyds mit einem Phenol der allgemeinen Formel

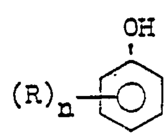

(II)

worin R und n die vorstehend angegebenen Bedeutungen haben, wovon wenigstens eine der beiden Stellungen in ortho zur Hydroxylgruppe frei ist, in Anwesenheit eines metallischen Katalysators und gegebenenfalls einer Base, dadurch gekennzeichnet, daß man als Katalysator ein Derivat des Titan (IV) verwendet.

2. Verfahren gemäß Anspruch 1, dadurch gekennzeichnet, daß das Derivat des Titans eine in dem Reaktionsmilieu lösliche Verbindung ist, die mit dem eingesetzten Phenol und dem Formaldehyd unter den Reaktionsbedingungen Komplexe zu bilden vermag.

3. Verfahren gemäß Anspruch 2, dadurch gekennzeichnet, daß der Metallkatalysator ein Salz von Mineralsäuren, ein Alkoholat, ein Phenolat, ein Salz von Carbon- oder Sulfonsäuren, ein Komplex des Titans mit einer Verbindung, die ein Donor von elektronischem Dublett ist, ist.

4. Verfahren gemäß Anspruch 3, dadurch gekennzeichnet, daß der metallische Katalysator ausgewählt ist aus der Gruppe, bestehend aus Titantetraphenoxid, Titantetrabutoxid, Titantetratolyloxid, Dichlordicyclopentadienyltitan, Tetrakis-(dimethylamino)-titan.

5. Verfahren gemäß einem der Ansprüche 1 bis 4, dadurch gekennzeichnet, daß die Katalysatormenge, ausgedrückt im Gramm-Atom Metallionen je Mol Phenol, zwischen 0,0001 und 1 Gramm-Atom Metallion je Mol Phenol liegt.

6. Verfahren gemäß einem der Ansprüche 1 bis 5, dadurch gekennzeichnet, daß das Verfahren in Anwesenheit einer Base durchgeführt wird, ausgewählt aus der Gruppe der tertiären Stickstoffbasen und ihrer N-Oxide, der Phosphine, Arsine, Stibine und Phosphotriamide.

7. Verfahren gemäß Anspruch 6, dadurch gekennzeichnet, daß die Base Pyridin, Triphenylphosphin, N-Oxypyridin oder Hexamethylphosphotriamid ist.

8. Verfahren gemäß einem der Ansprüche 1 bis 7, dadurch gekennzeichnet, daß die Menge der eingesetzten Base, ausgedrückt in Mol je Mol phenolisches Substrat, zwischen 0,001 und 1,5 Mol je Mol Phenol variiert.

9. Verfahren gemäß einem der Ansprüche 1 bis 8, dadurch gekennzeichnet, daß das Verfahren in einem inerten, organischen, aprotischen, nicht- oder schwach polaren Lösungsmittel durchgeführt wird.

10. Verfahren gemäß Anspruch 9, dadurch gekennzeichnet, daß das Lösungsmittel ausgewählt ist aus der Gruppe, gebildet von den gesättigten, aliphatischen und cycloaliphatischen Kohlenwasserstoffen, den aromatischen Kohlenwasserstoffen, den halogenierten Kohlenwasserstoffen, den Estern und den Ethern.

11. Verfahren gemäß einem der Ansprüche 1 bis 8, dadurch gekennzeichnet, daß die eingesetzten Phenole sind : das Phenol, das meta-Cresol, das para-Cresol, das Guajacol, das p-Chlorphenol, das p-Vanillin.